(19) **Europäisches Patentamt**
**European Patent Office**
**Office européen des brevets**

(11) **EP 2 282 048 A1**

(12) **EUROPÄISCHE PATENTANMELDUNG**

(43) Veröffentlichungstag:
**09.02.2011 Patentblatt 2011/06**

(21) Anmeldenummer: **09008697.6**

(22) Anmeldetag: **02.07.2009**

(51) Int Cl.:
*F03B 13/14* (2006.01)          *E02B 9/08* (2006.01)
*G01N 33/574* (2006.01)

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(71) Anmelder: **Bayer MaterialScience AG**
**51368 Leverkusen (DE)**

(72) Erfinder: **Die Erfindernennung liegt noch nicht vor**

(54) **Verfahren Gewinnung von elektrischer Energie aus der Bewegungsenergie von Wasserwellen**

(57) Die vorliegende Erfindung betrifft ein Verfahren zur Gewinnung von elektrischer Energie aus der Bewegungsenergie von Wasserwellen. In dem Verfahren wird eine Vorrichtung im Wasser bereitgestellt, welche ein durch Einwirkung der Wasserwellen dehnbares elektroaktives Polymer umfasst. Beim Dehnen des elektroaktiven Polymers wird zu einem bestimmten Zeitpunkt für ein bestimmtes Zeitintervall eine elektrische Ladung beaufschlagt. Diese wird beim Relaxieren des Polymers bis auf eine Restladung entnommen. Im Verfahren werden die für den Betrieb des Verfahrens benötigten Größen der elektrischen Zielladung und der Zeitpunkte für Beginn und Ende des Ladens und Entladens des elektroaktiven Polymers ermittelt. Die Erfindung betrifft weiterhin ein System zur Gewinnung von elektrischer Energie und ein Computerprogrammprodukt, umfassend durch einen Mikroprozessor ausführbare Befehle zur Durchführung der Berechnungen im erfindungsgemäßen Verfahren.

EP 2 282 048 A1

## Beschreibung

Die vorliegende Erfindung betrifft ein Verfahren zur Gewinnung von elektrischer Energie aus der Bewegungsenergie von Wasserwellen. In dem Verfahren wird eine Vorrichtung im Wasser bereitgestellt, welche ein durch Einwirkung der Wasserwellen dehnbares elektroaktives Polymer umfasst. Beim Dehnen des elektroaktiven Polymers wird zu einem bestimmten Zeitpunkt für ein bestimmtes Zeitintervall eine elektrische Ladung beaufschlagt. Diese wird beim Relaxieren des Polymers bis auf eine Restladung entnommen. Im Verfahren werden die für den Betrieb des Verfahrens benötigten Größen der elektrischen Zielladung und der Zeitpunkte für Beginn und Ende des Ladens und Entladens des elektroaktiven Polymers ermittelt.

Die Energiegewinnung aus Wasserwellen mittels elektroaktiven Polymergeneratoren (EAP-Generatoren) basiert auf dem Prinzip der Umwandlung von potenzieller Wellenenergie in eine Dehnung des EAP-Generators in einer ersten Stufe, die dann unter Verwendung eines Energiegewinnungszyklus (Energy Harvesting Cycle, EHC) schließlich in elektrische Energie umgewandelt wird.

Beispiele für elektromechanische Wandler finden sich in WO 2001/06575 A1. Diese Patentanmeldung betrifft Wandler, ihre Verwendung und ihre Herstellung. Solch ein Wandler zur Umwandlung von mechanischer Energie in elektrische Energie umfasst wenigstens zwei Elektroden und ein Polymer. Das Polymer ist so angeordnet, dass als Folge einer Längenänderung eines ersten Abschnittes ein elektrisches Feld verändert wird. Weiterhin ist ein zweiter Abschnitt des Polymers elastisch vorgespannt.

WO 2007/130252 A2 offenbart Systeme und Verfahren, welche einen EAP-Wandler zur Umwandlung von ursprünglich in einer oder mehrer Wellen enthaltenen mechanischen Energie einsetzen. Im Meer einsetzbare Vorrichtungen können ein mechanisches Energieumwandlungssystem verwenden, welche mechanische Energie übertragen, welche zum Einwirken auf den EAP-Wandler geeignet ist. Solch eine marine Vorrichtung umfasst einen Körper, wobei ein Teil des Körpers sich oberhalb der Wasseroberfläche befindet, wenn die Vorrichtung im Wasser schwimmt. Die Vorrichtung umfasst weiterhin ein System zur Übertragung mechanischer Energie aus einer Veränderung des Niveaus der Wasseroberfläche in durch das System regulierte mechanische Energie. Ein EAP-Wandler ist an einen Teil des Systems zur Übertragung mechanischer Energie gekoppelt und eingerichtet, um elektrische Energie aus der regulierten mechanischen Energie in dem System zur Übertragung mechanischer Energie zu erzeugen. Der EAP-Wandler weist ein elektroaktives Polymer und wenigstens zwei an das elektroaktive Polymer gekoppelte Elektroden auf.

Die Optimierung des Energiegewinnungszyklus stellt neben der mechanisch geeigneten Anordnung eine wesentliche Möglichkeit zur Steigerung der Energieeffizienz von EAP-Generatoren dar. Insbesondere trägt in diesem Zusammenhang das Steuerungs- beziehungsweise Regelungskonzept für die Lade-/Entladevorgänge einen wichtigen Beitrag bei.

Unter theoretischen Gesichtspunkten ist zur optimalen Wandlungseffizienz von EAP-Generatoren folgender Zyklus anzustreben, welcher einen theoretischen Grenzzyklus darstellt:

1. Phase: der EAP-Generator wird auf Grund einer äußeren Krafteinwirkung gedehnt. Diese Kraft wird zum Beispiel durch Ausnutzung der Änderung von potenzieller Energie hervorgerufen, wie sie bei Wellenbewegungen auftritt.

2. Phase: bei maximaler Dehnung des EAP-Generators wird die Anordnung, die aus einem zwischen zwei nachgiebigen Elektroden eingebrachten Polymer besteht, mit elektrischen Ladungen beaufschlagt. Die unter elektrischen Gesichtspunkten als Kondensator mit veränderlicher Kapazität zu betrachtende Anordnung wird bis zum Erreichen der elektrischen Durchbruchsfeldstärke geladen.

3. Phase: bei einer Verringerung der äußeren angreifenden Kraft relaxiert das EAP wegen der im Polymer wirkenden elastischen Rückstellkräfte. Bei diesem Vorgang erhöht sich die im EAP-Generator gespeicherte elektrische Energie. Diese Phase beschreibt den Energiewandlungsprozess von mechanischer in elektrische Energie.

4. Phase: sobald die elastischen Rückstellkräfte innerhalb des Polymers betragsmäßig gleich der entgegengesetzt gerichteten Kraft sind, die durch den elektrostatischen Druck des geladenen Kondensators hervorgerufen werden, zieht sich das EAP nicht weiter zusammen und die Anordnung sollte entladen werden. Nach diesem Prozess hat der EAP-Generator wieder seine Ausgangslänge auf Grund des sich verringernden elektrostatischen Drucks erreicht.

Der zuvor beschriebene theoretische Zyklus kann unter praktischen Gesichtspunkten nur annähernd durchlaufen werden. Der theoretisch anzustrebende Betrieb an der elektrischen Durchbruchsfeldstärke kann in der Praxis zur Zerstörung der Anordnung führen. Aus diesem Grund muss die anliegende Feldstärke zu jedem Zeitpunkt des Energiegewinnungszyklus unterhalb der elektrischen Durchbruchsfeldstärke liegen. Je höher allerdings die Feldstärke gewählt werden kann, desto höher ist der Energiegewinn des EAP-Generators.

Damit möglichst viel elektrische Energie gewonnen werden kann, sollte die beaufschlagte Ladung folglich möglichst nahe an der Durchbruchfeldtärke liegen. Ein fest vorgegebener Wert für die beaufschlagte Ladung ist jedoch nachteilig, wenn die Amplitude der Wellen sich ändert. Mit einer geringeren Wellenhöhe geht eine geringere Dehnung des elektroaktiven Polymers einher, so dass das Aufladen des Polymers nicht mehr optimal ablaufen kann. Im praktischen Betrieb ist weiterhin zu berücksichtigen, dass der Ladestrom für das Polymer nicht unendlich hoch ist, die Aufladezeit also nicht unendlich klein ist. Vielmehr wird über einen gewissen Zeitraum die Ladung auf das Polymer übertragen. Entsprechendes gilt für das Entladen des Polymers. In der Optimierung der Energiegewinnung müssen auch diese Zeiträume hinsichtlich Beginn, Ende und Dauer mit berücksichtigt werden.

Wünschenswert wäre daher eine Verbesserung der Annäherung des praktisch durchführbare Energiewandlungszyklus an den optimalen Zyklus. Dieses beinhaltet ein Steuerungsbeziehungsweise Regelungskonzept zur Realisierung des Lade-/Entladevorgangs.

Erfindungsgemäß vorgeschlagen wird ein Verfahren zur Gewinnung von elektrischer Energie aus der Bewegungsenergie von Wasserwellen, umfassend die Schritte:

Bereitstellen einer Vorrichtung im Wasser, wobei die Vorrichtung so eingerichtet ist, dass ein Teil der Vorrichtung oberhalb der Wasseroberfläche angeordnet ist, wobei die Vorrichtung ein elektroaktives Polymer umfasst, welches zwischen Elektroden angeordnet ist und wobei in der Vorrichtung mechanische Energie aus einer Veränderung des Wasserspiegels auf das elektroaktive Polymer übertragen wird;

Dehnen des elektroaktiven Polymers von einer minimalen Dehnung $\varepsilon_{min}$ bis zu einer maximalen Dehnung $\varepsilon_{max}$ als Folge einer mechanischen Einwirkung einer Wasserwelle mit einer Ausbreitungsgeschwindigkeit c und einer Wellenlänge $\lambda$,
wobei während des Dehnens des elektroaktiven Polymers dieses während eines Zeitintervalls $\Delta t_{load}$ von einem Zeitpunkt $t_1$ bis zu einem Zeitpunkt $t_{load}$ mit einer elektrischen Zielladung $Q^*_{load}$ beaufschlagt wird, bei der die elektrische Durchbruchsfeldstärke $E_{max}$ des elektroaktiven Polymers nicht überschritten wird;

Relaxieren des gedehnten elektroaktiven Polymers von der maximalen Dehnung $\varepsilon_{max}$ bis zu der minimalen Dehnung $\varepsilon_{min}$ als Folge einer nachlassenden mechanischen Einwirkung der Wasserwelle,
wobei während des Relaxierens des elektroaktiven Polymers dieses während eines Zeitintervalls $\Delta t_{unload}$ von einem Zeitpunkt $t_2$ bis zu einem Zeitpunkt $t_{unload}$ bis auf eine Restladung $Q_0$ entladen wird; und

wobei zur Berechnung der elektrischen Zielladung erforderliche Zahlenwert der maximalen Dehnung $\varepsilon_{max}$ im Voraus abgeschätzt wird durch:

Abschätzen der maximalen Wellenhöhe $h_{max}$ einer Wasserwelle und

Korrelieren der maximalen Wellenhöhe $h_{max}$ mit der maximalen Dehnung $\varepsilon_{max}$ über eine Zuordnungsvorschrift.

Elektroaktive Polymere (EAP) im Sinne der vorliegenden Erfindung sind grundsätzlich Polymere, die durch das Anlegen einer elektrischen Spannung ihre Form ändern und insbesondere dielektrische Elastomere. Geeignete Materialien sind insbesondere dielektrische Polyurethan-Elastomere. Das elektroaktive Polymer kann mit zwei oder mehr Elektroden verbunden sein, welche ebenfalls dehnbar sein können. Zweckmäßigerweise wird das erfindungsgemäße Verfahren im Meer durchgeführt, die Vorrichtung also im Meer bereitgestellt. Die Vorrichtung kann allgemein als elektromechanischer Wandler bezeichnet werden.

Im erfindungsgemäßen Verfahren wird das gedehnte elektroaktive Polymer mittels einer geeigneten Einheit, welche beispielsweise als Leistungselektronik oder Ladeelektronik bezeichnet werden kann, mit einer elektrischen Ladung beaufschlagt. Beim Relaxieren oder Entspannen wird diese Ladung wieder entnommen, wodurch elektrische Energie gewonnen wird.

Zur Berechnung der möglichst hohen elektrischen Zielladung $Q^*_{load}$ ist die Kenntnis über die maximale Dehnung $\varepsilon_{max}$ erforderlich, wie weiter unten näher ausgeführt werden wird. Da der Zeitpunkt des Endes des Beaufschlagens der Zielladung, $t_{load}$, um das Maximum der Dehnung $\varepsilon_{max}$ und somit um das Maximum der Wellenhöhe liegt, muss der Zeitpunkt des Beginns des Beaufschlagens, $t_1$, zwangsläufig vor dem Maximum liegen. Entsprechendes gilt für das Entladen des Polymeres. Es ist also nötig, vorherzusagen, wann das Maximum der Welle den elektromechanischen Wandler erreicht und wie hoch das Maximum ausfallen wird.

Im erfindungsgemäßen Verfahren wird zunächst die zu erwartende maximale Wellenhöhe für die nächste Welle abgeschätzt. Dieses wird ebenfalls weiter unten näher erläutert. Der benötigte Zahlenwert für die maximale Dehnung $\varepsilon_{max}$ wird über eine Korrelation mit der maximalen Wellenhöhe $h_{max}$ erhalten. Die Zuordnungsvorschrift kann beispielsweise eine empirisch ermittelte Korrelationstabelle sein, welche jeder Auslenkung des elektromechanischen Wandlers eine Dehnung zuordnet. Die Vorschrift kann auch eine mathematische Beschreibung der speziellen Wandlermechanik sein, also ein Modellkonzept sein. Eine weitere Möglichkeit basiert auf Messdaten des elektroaktiven Polymers.

Die im erfindungsgemäßen Verfahren ermittelten Zeitpunkte für das Laden und Entladen des elektroaktiven Polymers können mit einer internen Zeitmessung korreliert werden, um auf einer absoluten Skala das Polymer ansteuern zu können. Es ist auch möglich, externe Zeitsignale wie beispielsweise die über Langwelle gesendeten Signale von Atomuhren zu verwenden.

Das erfindungsgemäße Verfahren bietet insgesamt den Vorteil, dass auf eine Veränderung der Wellenhöhe im Laufe der Zeit reagiert werden kann und somit auch unter wechselnden Bedingungen näher am Optimum der Energiegewinnung gearbeitet werden kann.

Die vorliegende Erfindung wird unter Bezugnahme auf die nachfolgenden Zeichnungen näher beschrieben werden. Hierbei zeigen:

FIG. 1     eine beispielhafte Überlagerung eines Wellenverlaufs mit dem Lade- und Entladestrom des EAP
FIG. 2     ein Ablaufdiagramm für eine Berechnung gemäß dem erfindungsgemäßen Verfahren
FIG. 3     ein Blockschaltbild für ein erfmdungsgemäßes System

Zunächst soll zum besseren Verständnis auf die Theorie der Wasserwellen eingegangen werden. Bei Wasserwellen wird grundsätzlich unterschieden in Flachwasser- und Tiefwasserwellen. Flachwasserwellen treten in Strandnähe beziehungsweise geringer Wassertiefe $d$ auf. Die Wellenlänge ist hier deutlich länger als die Wassertiefe: $\lambda \gg d$. Tiefwasserwellen entstehen auf dem offenen Meer mit $\lambda \ll d$ und eignen sich im besonderen Maße für die Energiegewinnung.

In der Regel breiten sich Tiefwasserwellen sinusförmig auf dem Meer aus. Frequenz und Ausbreitungsgeschwindigkeit ändern sich nur sehr langsam. Die Höhe der Wellen ist Rayleighverteilt, das heißt, große Wellenhöhen treten selten auf. Die größte Wahrscheinlichkeit liegt bei kleineren Amplituden.

Zur Steuerung des Laderzyklus für den EAP-Generator muss lediglich die Grundwelle der Tiefwasserwelle (Transversalwelle) betrachtet werden. Diese wird beschrieben durch die Wellenlänge $\lambda$, die Wassertiefe $d$, mit der orts- und zeitabhängigen Wellenhöhe $h(x,t)$ und ihrer maximalen Wellenhöhe $h_{\max}$ sowie durch die Ausbreitungsgeschwindigkeit c. Die Wellenhöhe ergibt sich wie in Gleichung (1) dargestellt:

$$h(x,t) = h_{\max} \cdot \cos\left( \frac{2 \cdot \pi}{\lambda} \cdot x - \frac{2 \cdot \pi}{\lambda} \cdot c \cdot t \right) \qquad (1)$$

Bezogen auf eine ganze Welle der Länge $\lambda$ ergibt sich die auf die Fläche $A$ bezogene kinetische Energie gemäß Gleichung (2):

$$\frac{\overline{E_{kin}}}{A} = \frac{1}{4} \rho \cdot c^2 \cdot \frac{h_{\max}^2}{d} \qquad (2)$$

Bezogen auf eine ganze Welle der Länge $\lambda$ ergibt sich die auf die Fläche $A$ bezogene potenzielle Energie gemäß Gleichung (3):

$$\frac{\overline{E_{pot}}}{A} = \frac{1}{4} \cdot \rho \cdot g \cdot h_{\max}^2 \qquad (3)$$

Die auf die Fläche $A$ bezogene potenzielle Energie, die zur Dehnung des EAP-Generators verwendet werden soll, ist abhängig von der maximalen Wellenhöhe $h_{\max}$, der Erdbeschleunigung g und der Dichte des Wassers $\rho$.

Gegenüber der kinetischen Energie in Gleichung (2) verändert sich die flächenbezogene potenzielle Energie ausschließlich mit der Wellenhöhe, so dass durch Gleichsetzen sich schließlich die Ausbreitungsgeschwindigkeit c der Welle bestimmen lässt:

$$\begin{aligned}
\frac{\overline{E}_{pot}}{A} &= \frac{\overline{E}_{kin}}{A} \\
\frac{1}{4} \cdot \rho \cdot g \cdot h_{max}^2 &= \frac{1}{4} \rho \cdot c^2 \cdot \frac{h_{max}^2}{d} \\
c &= \sqrt{g \cdot d}
\end{aligned} \qquad (4)$$

Die Ausbreitungsgeschwindigkeit der Welle ist gemäß Gleichung (4) lediglich eine Funktion der Erdbeschleunigung g und der Wassertiefe $d$. Letztere ändert sich (bei fester Position des EAP-Generators) nur geringfügig auf Grund der Gezeiten, so dass die Ausbreitungsgeschwindigkeit näherungsweise konstant ist.

Mit der in der Wasserwellentheorie begründeten Annahme einer sinusförmigen Ausbreitung von Tiefwasserwellen soll im folgenden Abschnitt die Optimierung des Energiegewinnungszyklus beschrieben werden. Ziel ist hierbei die Bestimmung der zeitlich optimalen Lade- und Entladezyklen und die Bestimmung der jeweils maximal aufzubringenden Ladungsmenge unter Einhaltung von Randbedingungen.

Auf Grund des durch die Elektronik begrenzten Laderstroms und der damit endlichen Zeit zum Aufbringen von Ladungen auf den EAP-Generator ist unter Bestimmung der optimalen Lade- und Entladezyklen die Detektierung des optimalen Lade-/Entladezeitintervalls zu verstehen. Unter Berücksichtigung der im Zyklus auftretenden maximalen Dehnung des EAP soll die übertragene Ladungsmenge, die über die Kapazität des EAP mit der elektrischen Spannung in Relation steht, so vorausbestimmt werden, dass die elektrische Durchbruchsfeldstärke $E_{max}$ des Polymers einerseits zu keinem Zeitpunkt überschritten wird, aber andererseits zu maximaler Energiegewinnung führt.

Für die Optimierung des Energiegewinnungszyklus sollen zwei Kriterien für die Extremalberechnung betrachtet werden, nämlich die Maximierung der Energiewandlung von mechanischer in elektrische Energie und die Minimierung elektrischer und mechanischer Verluste.

Zunächst sollen die Optimierungsbedingungen für eine maximale Energiegewinnung allgemein hergeleitet werden.

Soll das EAP zum Zeitpunkt $t_1$ mit dem Ladestrom $i_{load}(t)$ in der Ladezeit $\Delta t_{load} = t_{load} - t_1$ auf eine vorab bestimmte Zielladung $Q^*_{load}$ geladen werden, ergibt sich die zeitabhängige Ladungsmenge zu:

$$Q^*_{load} = \int_{t_1}^{t_{load}} i_{load}(t)\,dt \qquad (5)$$

Mit Vorgabe von $Q^*_{load}$, und bei bekanntem $i_{load}(t)$ lässt sich aus Gleichung (5) das Ladezeitintervall $\Delta t_{load} = t_{load} - t_1$ bestimmen. Die Bestimmung des Entladezeitintervalls folgt analog.

Die Kapazität C des EAP-Generators ist lediglich von der Dehnung $\varepsilon$ abhängig, die wiederum eine Funktion des Ortes und der Zeit der Wasserwelle ist, aber keine Funktion der EAP-Spannung $u_{EAP}$ ist.

Allgemein beträgt die auf den EAP-Generator aufgebrachte Energie nach Abschluss des Ladezyklus:

$$E_{load} = \int_{t_1}^{t_1+\Delta t_{load}} u_{EAP}(t) \cdot i_{load}(t)\,dt = \int_{t_1}^{t_1+\Delta t_{load}} \frac{Q(t)}{C(\varepsilon(x,t))} \cdot i_{load}(t)\,dt \qquad (6)$$

Die vom EAP-Generator entnommene Energie nach Abschluss des Entladevorgangs beträgt:

$$E_{unload} = \int_{t_2}^{t_2+\Delta t_{unload}} u_{EAP}(t) \cdot i_{unload}(t)\,dt = \int_{t_2}^{t_2+\Delta t_{unload}} \frac{Q(t)}{C(\varepsilon(x,t))} \cdot i_{unload}(t)\,dt \qquad (7)$$

Der Energiegewinn ergibt sich dann zu:

$$E_{harvest} = E_{unload} - E_{load} \tag{8}$$

Aus dieser allgemeinen Gleichung für den Energiegewinn können die optimalen Startpunkte $t_1$ und $t_2$ durch Lösung der Extremwertaufgabe

$$\max\left(E_{harvest}(t_1,t_2)\right) \text{ mit } t_2 > t_1 \text{ und } t_1+t_2 < \lambda/c \tag{9}$$

bestimmt werden. Da der Energiegewinn von zwei Variablen abhängt, können Extremwerte nur auftreten, wenn die partiellen Ableitungen nach den Variablen $t_1$, $t_2$ gleichzeitig Null sind:

$$\frac{\partial E_{harvest}(t_1,t_2)}{\partial t_1} = 0, \qquad \frac{\partial E_{harvest}(t_1,t_2)}{\partial t_2} = 0 \tag{10}$$

Die Lösung von Gleichung (10) ergibt Wertepaare, die Extremwerte der Flächenfunktion in Gleichung (10) darstellen. Ob es sich hierbei um ein Minimum, Maximum oder Sattelpunkt handelt, kann mit den zweiten Ableitungen bestimmt werden:

$$\frac{\partial^2 E_{harvest}(t_1,t_2)}{\partial t_1^{\,2}} = 0, \quad \frac{\partial^2 E_{harvest}(t_1,t_2)}{\partial t_2^{\,2}} = 0, \quad \frac{\partial^2 E_{harvest}(t_1,t_2)}{\partial t_1 \partial t_2} = 0, \quad \frac{\partial^2 E_{harvest}(t_1,t_2)}{\partial t_2 \partial t_1} = 0 \tag{11}$$

Liefern die beiden gemischten Ableitungen nach $t_1$, $t_2$ identische Ergebnisse, so liegt ein Maximum vor, wenn die beiden zweiten Ableitungen nach $t_1$ oder $t_2$ negativ sind; ein Minimum liegt vor, wenn die beiden zweiten Ableitungen positiv sind; ein Sattelpunkt liegt vor, wenn die beiden zweiten Ableitungen unterschiedliche Vorzeichen aufweisen.
Unabhängig vom Ergebnis der beiden gemischten Ableitungen kann die Entscheidung, welches Extremum vorliegt, allgemein mit Hilfe der Hesse-Matrix vorgenommen werden:

$$\mathbf{H} = \begin{bmatrix} \dfrac{\partial^2 E_{harvest}(t_1,t_2)}{\partial t_1^{\,2}} & \dfrac{\partial^2 E_{harvest}(t_1,t_2)}{\partial t_1 \partial t_2} \\[2em] \dfrac{\partial^2 E_{harvest}(t_1,t_2)}{\partial t_2 \partial t_1} & \dfrac{\partial^2 E_{harvest}(t_1,t_2)}{\partial t_2^{\,2}} \end{bmatrix} \tag{12}$$

Ist die Matrix H positiv definit, liegt ein relatives Minimum vor, ist H negativ definit handelt es sich um ein relatives Maximum, ist H indefinit, so handelt es sich um kein relatives Extremum. Sofern **H** positiv/negativ semidefinit ist, sind zusätzliche Untersuchungen notwendig, wie sie in üblichen Lehrbüchern der Mathematik näher erläutert werden.
In einer Ausführungsform des erfindungsgemäßen Verfahrens wird der Zeitpunkt $t_1$ berechnet gemäß

$t_1 = \dfrac{\lambda}{4 \cdot c} - \Delta t_{load}$ und der Zeitpunkt $t_2$ wird gemäß $t_2 = 3 \cdot \dfrac{\lambda}{4 \cdot c}$ berechnet. Weiterhin hat zu dem Zeitpunkt $t_{load}$ das elektroaktive Polymer die maximale Dehnung $\varepsilon_{max}$ eingenommen und zu dem Zeitpunkt $t_{unload}$ das elektroaktive Polymer die minimale Dehnung $\varepsilon_{min}$.

Unter der Annahme einer sinusförmigen Ausbreitung der Wasserwelle ist bei ideal elastischen Verhältnisses des Polymers auch ein annähernd sinusförmiger Dehnungsverlauf $\varepsilon$ des EAP-Generators zu erwarten. Die Kapazität des EAP-Generators wird bei steigender Dehnung einen streng monoton steigenden Verlauf, bei Relaxation einen streng monoton fallenden Verlauf aufweisen und kann in erster Näherung ebenfalls als sinusförmiger Verlauf approximiert werden. Demnach weist die Anordnung bei maximaler Dehnung $\varepsilon_{max}$ die maximale Kapazität und $C_{max}$ und bei minimaler

Dehnung $\varepsilon_{min}$ die minimale Kapazität $C_{min}$ auf.

Ändert sich die Kapazität des EAP-Generators konstant proportional zur Dehnung $\varepsilon$ des EAP-Generators

$$C(t) = C_0 + C_{max} \cdot \sin\left(2 \cdot \pi \cdot \frac{c}{\lambda} \cdot t\right) \qquad (13)$$

und sind die Lade- und Entladeströme auf Grund ihres Begrenzungswertes $I_{max}$ blockförmig, womit auch

$$Q(t) = I_{max} \cdot (t - t_1) \text{ beziehungsweise } Q(t) = -I_{max} \cdot (t - t_2) \qquad (14)$$

gilt, ergibt sich der maximale Energiegewinn nach Gleichungen (9)-(12) genau dann, wenn bei der maximalen Amplitude der Dehnung der Ladevorgang abgeschlossen ist, was auf den Startzeitpunkt

$$t_1 = \frac{\lambda}{4 \cdot c} - \Delta t_{load} \qquad (15)$$

führt und wenn der Entladevorgang bei minimaler Amplitude der Dehnung bei

$$t_2 = 3 \cdot \frac{\lambda}{4 \cdot c} \qquad (16)$$

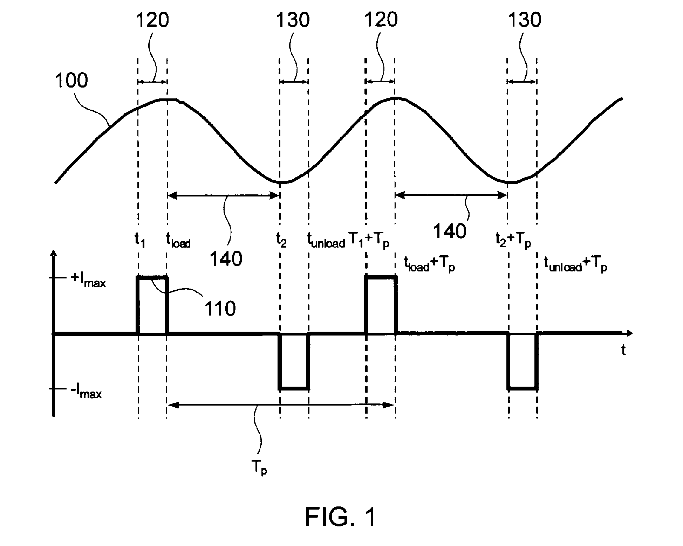

beginnt. Der Lade- und Entladevorgang wiederholt sich nach einer Periodendauer $T_p = \lambda/c$. Der Zusammenhang wird nochmals in FIG. 1 dargestellt.

FIG. 1 zeigt für diese Ausführungsform schematisch die Überlagerung des Verlaufs einer Wasserwelle 100 mit dem Lade- und Entladestrom 110 des elektroaktiven Polymers. Zum Zeitpunkt $t_1$ beginnt das Aufladen des Polymers bis zum Endzeitpunkt $t_{load}$, welcher mit dem Maximum der Wellenhöhe zusammenfällt. Dieses Ladeintervall ist durch das Bezugszeichen 120 gekennzeichnet. Vom Zeitpunkt $t_2$, welcher mit dem Minimum der Wellenhöhe korrespondiert, bis zum Zeitpunkt $t_{unload}$ wird das Polymer entladen. Dieses ist das Entladeintervall 130. Die Energiewandlung findet jeweils im Intervall 140 statt, wenn das aufgeladene EAP wieder relaxiert.

Der Energiegewinn $E_{harvest}$ durch den EAP-Generator wird durch Verluste reduziert. Es treten neben mechanischen Umwandlungsverlusten $E_{EAP.mech}$ und elektrischen Verlusten im EAP-Generator $E_{EAP,el}$ zusätzliche Verluste $E_{elec}$ durch die Ladeelektronik während der Lade- und Entladezyklen in Abhängigkeit der EAP-Spannung u(t) und der EAP-Kapazität $C(\varepsilon(x,t))$ auf, die bei der Optimierung des maximalen Energiegewinns berücksichtigt werden und einen Einfluss auf die optimalen Schaltzeitpunkte haben. Insofern ist bei der Zeitintervalloptimierung nach den Gleichungen (9)-(12) die nachfolgende Beziehung zu verwenden:

$$\max(E_{harvest} - E_{loss}) \text{ mit } E_{loss} = E_{EAP,mech} + E_{EAP,el} + E_{elec} \qquad (17)$$

Mit einem geeigneten Sensor- und Beobachterkonzept werden die Zeitpunkte minimaler Amplitude $t(h_{min})$ und maximaler Amplitude $t(h_{max})$ und die Wellenhöhe $h_{max}$ vorhergesagt. Beispielsweise mit einem Modell des EAP-Generators und des mechanischen Wandlerkonzeptes können dann die entsprechenden Zeitpunkte minimaler und maximaler Dehnung sowie Dehnung des EAP-Generators bestimmt werden. Der Prädiktionshorizont (vorausgeschätzte Zeit $t_{estimate}$) muss dabei zwischen $0 < t_{estimate} < T_p = \lambda/c$, das heißt eine Periodedauer, betragen. Durch eine räumlich versetzte Anordnung zwischen Sensorik und EAP-Generator kann sich der Prädiktionshorizont entsprechend verlängern (zum Beispiel, wenn die Welle die verwendete Sensorik erst nach dem EAP-Generator erreicht) oder verkürzen (zum Beispiel, wenn die Welle die verwendete Sensorik vor dem EAP-Generator erreicht).

In Abhängigkeit der Wellenlänge und Ausbreitungsgeschwindigkeit ergibt sich die Periodendauer $T_p = \lambda/c$ der Welle für einen festen Ort x. Wenn sich ein erster EAP-Generator E1, bezogen auf die Ausbreitungsrichtung der Welle, vor einer Sensorik S befindet, erreicht ein Wellenberg den EAP-Generator E1 um die Zeit:

$$t_{E1} = \frac{x_{Sensorik} - x_{EAP}}{\lambda} \cdot T_p \tag{18}$$

vor der Sensorik und der Prädiktionshorizont $t_{estimate}$ muss um diesen Betrag größer werden. Befindet sich ein zweiter EAP-Generator E2 nach der Sensorik S, kann der Prädiktionshorizont $t_{estimate}$ entsprechend kleiner werden.

Die Schätzung der Wellenlänge $\lambda$ kann entweder mit einer zusätzlichen Sensorik erfolgen oder aus den elektrischen Größen des EAP-Generators bestimmt werden.

Wird zusätzliche Sensorik eingesetzt, bietet sich der Einsatz von Beschleunigungssensoren an, die die Vertikalbeschleunigung der Wasseroberfläche messen. Die Grundwelle kann zum Beispiel mit einer Phasenregelschleife (phase-locked loop, PLL) oder über Transformationen wie FourierTransformation und insbesondere Fast Fourier Transformationen (FFT) oder Cosinus-Transformation bestimmt werden.

Aus der Grundwelle und der Annahme, dass sich die Wellenlänge nur mit geringer Dynamik ändert, können die Zeitpunkte der Extremwerte der Wellenbewegung vorausgeschätzt werden.

Ein Beispiel für eine Schrittabfolge, welche noch zusätzliche Schritte umfassen kann, ist:

1. Schritt: Zunächst wird modell- oder/und sensorgestützt die Dehnung $\varepsilon$ und der Zeitpunkt maximaler Dehnung $t$ ($\varepsilon_{max}$) des EAP-Generators geschätzt.

2. Schritt: Anhand der zu erwartenden Dehnung des EAP-Generators wird modellbasiert die zulässige Zielladung $Q^*_{load}$ gemäß Gleichung (20) ermittelt, bei der ein Überschreiten der elektrischen Durchbruchsfeldstärke während des Energiegewinnungszyklus vermieden wird.

3. Schritt: Unter Berücksichtigung der Stromcharakteristik $i_{load}(t)$ kann die Länge der Ladezeit $\Delta t_{load}$ mit Gleichung (5) bestimmt werden. Analog wird die Länge der Entladezeit $\Delta t_{unload}$ bestimmt.

4. Schritt: Mittels der Zielladungsmenge $Q^*_{load}$ und der Lade- und Entladezeiten $\Delta t_{load}$ und $\Delta t_{unload}$ kann nun unter Berücksichtigung der Verluste $E_{loss}$ der optimale Zeitpunkt für den Beginn des Ladevorgangs $t_1$ und des Entladevorgangs $t_2$ nach Gleichung (17) bestimmt werden.

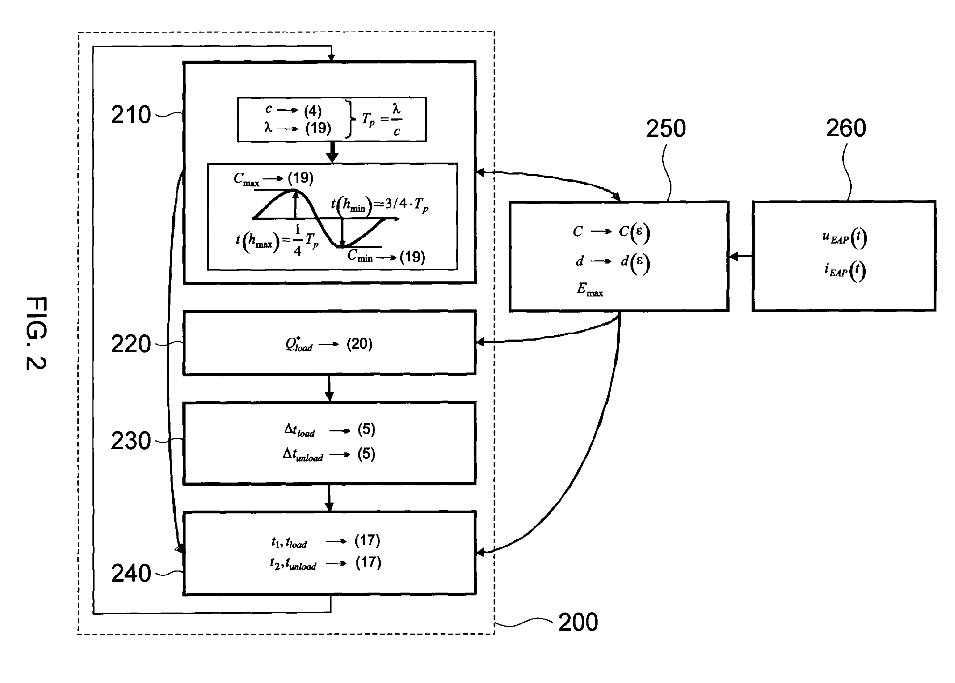

FIG. 2 zeigt ein Ablaufdiagramm für eine solche Schrittfolge. Hierbei bedeuten die Verweise durch Pfeile auf in Klammern gesetzte Zahlen, dass die Größe anhand der mit der entsprechenden Nummer referenzierten Gleichung in der Beschreibung zur vorliegenden Erfindung erhalten werden kann. Die eigentliche Schrittfolge ist in Gruppe 200 zusammengefasst. Schritt 260 betrifft das Ermitteln der zeitabhängigen Spannung $u_{EAP}(t)$ und des zeitabhängigen Stroms $i_{EAP}(t)$ des elektroaktiven Polymers. Aus diesen Werten können in Schritt 250 die dehnungsabhängige Kapazität $C(\varepsilon)$ und der dehnungsabhängige Elektrodenabstand $d(\varepsilon)$ bestimmt werden. Diese Werte dienen auch zur Vorhersage des Zeitpunktes der maximalen Dehnung des EAP in Schritt 210.

Unter Verwendung der vorhergesagten maximalen Dehnung des EAP werden in Schritt 220 die Zielladung $Q^*_{load}$ berechnet und in Schritt 230 die Zeitdauern für die Lade- und Entladeintervalle $\Delta t_{load}$ und $\Delta t_{unload}$. Schließlich können aus den ermittelten Parametern die Zeitpunkte für den Beginn des Ladevorgangs $t_1$ und des Entladevorgangs $t_2$ ermittelt werden.

In einer Ausführungsform des erfindungsgemäßen Verfahrens wird die Wellenlänge $\lambda$ ermittelt, indem mittels eines Beschleunigungssensors die vertikale Beschleunigung der Wasseroberfläche gemessen wird und:

die Oszillation des erhaltenen Beschleunigungssensor-Signals über eine Phasenregelschleife eine wellenlängenabhängige Spannung ergibt; und/oder

die Oszillation des erhaltenen Beschleunigungssensor-Signals über eine Fourier-oder Cosinus-Transformation in die Domäne c/$\lambda$, überführt wird.

Bevorzugte Fourier-Transformationen sind hierbei Fast Fourier-Transformationen.

In einer weiteren Ausführungsform des erfindungsgemäßen Verfahrens wird die Wellenlänge $\lambda$ ermittelt, indem die zwischen den Elektroden am elektroaktiven Polymer anliegende Spannung gemessen wird und:

die Oszillation der Spannung über eine Phasenregelschleife eine wellenlängenabhängige Spannung ergibt; und/oder

die Oszillation der Spannung über eine Fourier- oder Cosinus-Transformation in die Domäne c/λ überführt wird.

Bevorzugte Fourier-Transformationen sind hierbei Fast Fourier-Transformationen. Eine modellgestützte Schätzung ohne Sensorik besteht darin, am EAP-Generator die Spannung $u_{EAP}$ kontinuierlich zu messen, womit auf Basis der seitens der Steuerung bekannten Ladung $Q^*_{load} + Q_0$ beziehungsweise $Q_0$ des EAP-Generators über ein mathematisches Modell auf die Kapazität $C(\varepsilon(x,t))$ geschlossen werden kann. Damit die dehnungsabhängige EAP-Kapazität erfasst werden kann, muss beim Entladevorgang eine geringe Restladung $Q_0$ auf dem EAP verbleiben, um so eine fortlaufende Spannungsmessung während des Zyklus durchführen zu können.

$$C(\varepsilon(x,t)) = \begin{cases} \dfrac{Q_0 + \displaystyle\int_{t_1}^{t_{load}} i_{load}(t)\,dt}{u_{EAP}(t)} & \text{für } t_1 \leq t \leq t_{load} \\[3ex] \dfrac{Q^*_{load} + Q_0}{u_{EAP}(t)} & \text{für } t_{load} \leq t \leq t_2 \\[3ex] \dfrac{Q^*_{load} + \displaystyle\int_{t_1}^{t_{load}} i_{unload}(t)\,dt}{u_{EAP}(t)} & \text{für } t_2 \leq t \leq t_{unload} \\[3ex] \dfrac{Q_0}{u_{EAP}(t)} & \text{für } t_{unload} \leq t \leq t_1 \end{cases} \tag{19}$$

Über den formelmäßigen Zusammenhang zwischen EAP-Kapazität und EAP-Dehnung kann die zeitabhängige Dehnung des EAP-Generators bestimmt werden. Die Dehnung des EAP-Generators wird über das mechanische Wandlerkonzept durch die Wellenbewegung hervorgerufen, über den somit aus der EAP-Spannung auf die Wellenbewegung geschlossen werden. Durch Verwendung der Phasenregelschleife beziehungsweise Transformation der Wellenbewegung in den Frequenzbereich (analog zur Messung mit Beschleunigungssensor) kann die Wellenlänge bestimmt werden. Aus Wellenlänge und Ausbreitungsgeschwindigkeit leiten sich die Zeitpunkte minimaler und maximaler Dehnung $t(\varepsilon_{min})$, $t(\varepsilon_{max})$ ab.

Die Kenntnis über die Wellenhöhe ist insofern wichtig, damit die optimierte Ladungsmenge auf den EAP-Generator aufgebracht werden kann, die einen maximalen Energiegewinn erzeugt, aber nicht zur Zerstörung des EAP führt. Ist die Dehnung ε des EAP-Generators weitgehend unabhängig von der aufgebrachten Ladung ($\varepsilon \neq f(Q)$), wenn der elektrostatische Druck also vernachlässigt werden kann, berechnet sich die maximale Ladungsmenge über die maximal zulässige Feldstärke $E_{max}$ des EAP-Generators und den Elektrodenabstand $d$ (Polymerdicke). Dabei entsteht bei maximaler Dehnung $\varepsilon_{max}$ des EAP-Generators unter Annahme eines konstanten EAP-Volumens die maximale Kapazität $C_{max}$ und der minimale Elektrodenabstand $d_{min}$, während sich bei minimaler Dehnung $\varepsilon_{min}$ des EAP-Generators die minimale Kapazität $C_{min}$ und der maximale Elektrodenabstand $d_{max}$ einstellt. Da sowohl die Kapazität als auch der Elektrodenabstand nichtlineares Verhalten aufweisen, muss der gesamte Dehnungsbereich berücksichtigt werden und die zulässig aufzubringende Ladungsmenge $Q^*_{load}$ ergibt sich mit:

$$Q^*_{load} < \min\left[ C(\varepsilon(x,t)) \cdot E_{max} \cdot d(\varepsilon(x,t)) \right] \tag{20}$$

Wird während der Dehnung des EAP-Generators die Kapazität, wie mit Gleichung (18) vorgeschlagen, und in analoger Weise auch der Elektrodenabstand $d$ durch eine Spannungsmessung berechnet, kann aus deren geschätzten Verlauf die Zielladung $Q^*_{load}$ nach Gleichung (20) bestimmt werden. Eine Schätzung der Wellenhöhe ist daraus zwar ebenso möglich, allerdings für die optimale Steuerung des EHC nicht erforderlich.

Auf Basis der hergeleiteten Beziehungen sollten für jeden Zyklus der Zeitpunkt $t(\varepsilon_{max})$, $t(\varepsilon_{min})$ und die Amplitude der maximalen und minimalen Dehnung des EAP-Generators schon im Voraus ermittelt werden. Da sich jedoch insbesondere die Wellenlänge $\lambda$ nur langsam und in engen Grenzen ändert, muss lediglich die maximale Dehnung $\varepsilon_{max}$ in jedem Zyklus vorausgeschätzt werden.

In einer weiteren Ausführungsform des erfindungsgemäßen Verfahrens wird für den Fall, dass die tatsächliche Wellenhöhe sich gegenüber der geschätzten Wellenhöhe um so viel höher ist, dass mit der aufgebrachten Ladung die Durchschlagfeldstärke des elektroaktiven Polymers überschritten wird, aus dem elektroaktiven Polymer Ladung entnommen. Vorzugsweise wird für diesen Fall, dass $|E| \geq E_{max}$ ist, während des Energiegewinnungszyklus Ladung entnommen. Durch das Entnehmen der Ladung kann eine Zerstörung des EAP-Generators verhindert werden.

In einer weiteren Ausführungsform des erfindungsgemäßen Verfahrens ist die Vorrichtung:

eine Boje;

eine axial segmentierte Kette von fluidgefüllten Körpern aus einem ein elektroaktives Polymer umfassenden Material; oder

eine Anordnung von durch ein Drehgelenk miteinander verbundenen Schwimmkörpern mit exzentrisch angeordneten vorgespannten Abschnitten aus ein elektroaktives Polymer umfassendem Material, welche sich bei einer Knickbewegung der Schwimmkörper gegensätzlich zueinander dehnen und relaxieren.

Im Falle einer Boje folgt diese durch die ihre Auftriebskraft bei einem Wellenberg dem steigenden Wasserspiegel und erfährt eine Beschleunigung. An der Boje ist ein Zusatzgewicht angebracht. Auf Grund der Massenträgheit des Zusatzgewichts wird sich in dieser Phase eine Vergrößerung des Relativabstands zwischen Bojengehäuse und Zusatzgewicht einstellen, wodurch der EAP-Generator gedehnt wird.

Ebenso stellt sich bei der Bewegung der Boje ins Wellental eine Verringerung des Relativabstands zwischen Bojengehäuse und Zusatzgewicht ein. Für eine möglichst effiziente Ausnutzung des EAP-Generators ist der Zeitpunkt der minimalen Dehnung des Polymers erforderlich.

Bei quasistatischer Betrachtung findet in diesem Anwendungsfall das Laden der Anordnung bei einem Wellenberg statt. Das Entladen des EAP-Generators erfolgt dementsprechend im Wellental. Durch die mechanische Schwingungsanordnung können sich Phasenverschiebungen zwischen der Wellenoberfläche und der EAP-Dehnung ergeben, die bei Verwendung von Sensorik durch ein Wandlermodell berücksichtigt werden können.

Die axial segmentierte Kette von fluidgefüllten Körpern wird auch als "Anaconda"-Anordnung bezeichnet. Sie weist miteinander kommunizierende fluidgefüllte Körper aus einem ein elektroaktives Polymer umfassenden Material auf. Durch die Anhebung der Kette oder des Schlauches auf dem Wellenberg strömt das im Schlauch befindliche Fluid in die Wellentäler. Durch die geringe Steifigkeit des elektroaktiven Polymers wird der Querschnitt im Wellental erweitert (Dehnung) und auf dem Wellenberg (Relaxation) verjüngt.

Phasenverschiebungen zwischen der Wellenbewegung und der EAP-Dehnung, die sich auf Grund der Dynamik des im EAP befindlichen Fluids ergeben, können auch hier durch ein entsprechendes Wandlermodell im Algorithmus zur optimierten EHC-Steuerung berücksichtigt werden.

Die Anordnung von durch ein Drehgelenk miteinander verbundenen Schwimmkörpern wird auch als "Pelamis-Anordnung" bezeichnet. Sie ist so aufgebaut, dass zwei Schwimmkörper mit einem Drehgelenk verbunden sind. Exzentrisch integriert sind vorgespannte EAP, die sich bei der Knickbewegung der Pelamis-Anordnung gegensätzlich zueinander dehnen und relaxieren. Die Drehung im Gelenk wird dadurch erreicht, dass in beiden Schwimmkörper in räumlicher Nähe zum Gelenk Gewichte angebracht sind.

Auch dieses Wandlerkonzept findet gemäß den Erläuterungen zu den beiden Vorherigen in optimierten Steuerungsalgorithmen eine Berücksichtigung.

In einer weiteren Ausführungsform des erfindungsgemäßen Verfahrens umfasst das elektroaktive Polymer in der Vorrichtung ein Polyurethan, welches erhältlich ist aus der Reaktion eines isocyanatfunktionellen Prepolymers mit einem Polyetherpolyol. Das Prepolymer ist hierbei erhältlich aus der Reaktion eines Polyisocyanats mit einem mindestens trifunktionellen Polyetherpolyol, welcher eine Molekülmasse $M_n$ von $\geq 6000$ g/mol aufweist.

Geeignete Polyetherpolyole zur Reaktion mit dem Prepolymer sind insbesondere Polytetrahydrofuran mit einer Molekülmasse $M_n$ von $\geq 1000$ g/mol, vorzugsweise $\geq 2000$ g/mol und mehr bevorzugt $\geq 2500$ g/mol oder ein mittels Doppelmetallcyanid-Katalyse (DMC-Katalyse) hergestellter Polyether mit einer Molekülmasse $M_n$ von $\geq 2000$ g/mol, vorzugsweise $\geq 4000$ g/mol und mehr bevorzugt $\geq 6000$ g/mol.

Als Polyisocyanate zur Herstellung des Prepolymers werden bevorzugt solche aus der folgenden Reihe eingesetzt: Hexamethylendiisocyanat (HDI), Trimethyl-HDI, Isophorondiisocyanat (IPDI), Dodecahydro-MDI, Norbornandiisocyanat, Bisisocyanatomethylcyclohexan, Bisisocyanatomethylbenzol, alpha,alpha, alpha',alpha'-Tetramethylxylylendiisocyanat, 2,4-Toluylendiisocyanat (TDI) oder 2,6-TDI oder Gemische von 2,4- TDI und 2,6-TDI, des weiteren 2,2-Methylendiphenyldiisocyanat (MDI), 2,4-MDI oder 4,4-MDI oder Gemische von 2,2-MDI, 2,4-MDI und 4,4-MDI, des weiteren 3-Kern- oder Oligo-MDI-haltige MDI-Typen, wobei aliphatische Isocyanate besonders bevorzugt sind, des weiteren die Carbodiimide oder 4- oder 6-Ring-Heterocyclen enthaltenden Di- oder Trimere der vorgenannten Diisocyanate, oder Addukte der vorgenannten Diisocyanate an niedermolekulare Polyole, wie Trimethylolpropan, Diethylengylkol oder Dipropylenglykol, oder Urethan- oder Allophanat-Präpolymere der vorgenannten Diisocyanate an Polyole.

Gegebenenfalls können auch Füllstoffe zur Erhöhung der Dielektrizitätskonstante mit verwendet werden.

Ein weiterer Gegenstand der vorliegenden Erfindung ist ein System zur Gewinnung von elektrischer Energie aus der Bewegungsenergie von Wasserwellen und geeignet zur Durchführung des erfindungsgemäßen Verfahrens, umfassend:

eine Vorrichtung, welche so eingerichtet ist, dass beim Platzieren der Vorrichtung im Wasser ein Teil der Vorrichtung oberhalb der Wasseroberfläche angeordnet ist, wobei die Vorrichtung ein elektroaktives Polymer umfasst, welches zwischen Elektroden angeordnet ist und wobei in der Vorrichtung mechanische Energie aus einer Veränderung des Wasserspiegels auf das elektroaktive Polymer übertragen wird;

eine Sensoreinheit zur Gewinnung von Sensordaten über die Wasserwellen und/oder das elektroaktive Polymer;

eine Leistungseinheit zum Beaufschlagen des elektroaktiven Polymers mit elektrischer Ladung und zum Entladen des elektroaktiven Polymers; und

eine Steuereinheit, welche eingerichtet ist, um empfangene Daten aus der Sensoreinheit gemäß vorher definierter Datenmodelle in einem Berechnungsalgorithmus zu verarbeiten und als Ergebnis der Berechnung die Leistungselektronik anzusteuern, wobei weiterhin der Berechnungsalgorithmus die Abschätzung der maximalen Wellenhöhe von zukünftigen bei der Vorrichtung eintreffenden Wasserwellen umfasst.

Die Vorrichtung, welche ein Teil des erfindungsgemäßen Systems ist, entspricht der Vorrichtung im erfindungsgemäßen Verfahren. Daher wird zur Vermeidung von Wiederholungen auf die Erläuterungen hierzu verwiesen. Dieses gilt ebenfalls in Bezug auf bevorzugte Ausführungsformen der Vorrichtung.

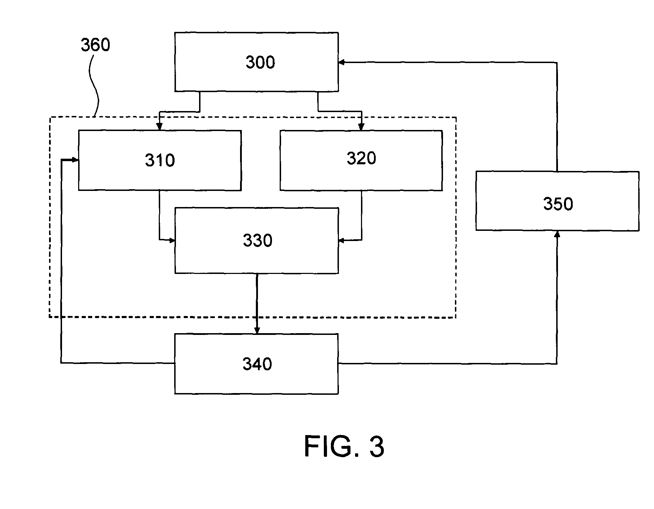

Ein erfindungsgemäßes System wird unter Bezugnahme auf FIG. 3 weiter erläutert, ohne jedoch darauf beschränkt zu sein. Diese Figur zeigt ein Blockschaltbild für ein erfindungsgemäßes System. Das Element 300 stellt die Sensoreinheit dar, welche Daten über die Wasserwellen und/oder das elektroaktive Polymer 350 gewinnt.

Die Daten aus der Sensoreinheit 300 können über ein mathematisches Modell und/oder ein mechanisches Konzept des elektromechanischen Wandlers 310 aufbereitet werden und dann an einen Berechnungseinheit 330 weitergegeben werden. Ebenso können die Daten in eine Modellierung der Wellen 320 einfließen. Die Berechnungseinheit 330 berechnet mit entsprechenden Algorithmen Vorgaben für eine Leistungselektronik oder Leistungseinheit 340. Solche Vorgaben sind insbesondere die auf das elektroaktive Polymer in der Vorrichtung zu übertragende Ladungsmenge und die Zeitpunkte für den Beginn und Ende des Ladens und Entladens. Die Steuereinheit 360 im erfindungsgemäßen System umfasst die Elemente 310, 320 und 330.

Durch die Leistungseinheit 340 wird das elektroaktive Polymer 350 mit einer elektrischen Ladung beaufschlagt und, nach Ende der Energiewandlungsphase, die elektrische Energie wieder entnommen. Zum Abgleich der Berechnungsmodelle kann die Leistungseinheit 340 mit der Modelleinheit 310 in Verbindung stehen.

In einer Ausführungsform des erfindungsgemäßen Systems umfasst die Sensoreinheit einen von der Vorrichtung beabstandeten Beschleunigungssensor. Dieser Beschleunigungssensor schwimmt vorzugsweise auf der Wasseroberfläche und misst die Schwankung deren Höhe als Folge der Wellenbewegungen. Details hierzu wurden bereits zum erfindungsgemäßen Verfahren erläutert.

In einer weiteren Ausführungsform des erfindungsgemäßen Systems misst die Sensoreinheit die zwischen den Elektroden am elektroaktiven Polymer anliegende Spannung und den Abstand der Elektroden am elektroaktiven Polymer. Details hierzu wurden bereits zum erfindungsgemäßen Verfahren erläutert.

Ein weiterer Gegenstand der vorliegenden Erfindung ist ein Computerprogrammprodukt, umfassend durch einen Mikroprozessor ausführbare Befehle zur Durchführung der Berechnungen in dem Verfahren gemäß der vorliegenden Erfindung.

**Patentansprüche**

1. Verfahren zur Gewinnung von elektrischer Energie aus der Bewegungsenergie von Wasserwellen, umfassend die Schritte:

Bereitstellen einer Vorrichtung im Wasser, wobei die Vorrichtung so eingerichtet ist, dass ein Teil der Vorrichtung oberhalb der Wasseroberfläche angeordnet ist, wobei die Vorrichtung ein elektroaktives Polymer umfasst, welches zwischen Elektroden angeordnet ist und wobei in der Vorrichtung mechanische Energie aus einer Veränderung des Wasserspiegels auf das elektroaktive Polymer übertragen wird;

Dehnen des elektroaktiven Polymers von einer minimalen Dehnung $\varepsilon_{min}$ bis zu einer maximalen Dehnung $\varepsilon_{max}$ als Folge einer mechanischen Einwirkung einer Wasserwelle mit einer Ausbreitungsgeschwindigkeit $c$ und einer Wellenlänge $\lambda$,

wobei während des Dehnens des elektroaktiven Polymers dieses während eines Zeitintervalls $\Delta t_{load}$ von einem Zeitpunkt $t_1$ bis zu einem Zeitpunkt $t_{load}$ mit einer elektrischen Zielladung $Q^*_{load}$ beaufschlagt wird, bei der die elektrische Durchbruchsfeldstärke $E_{max}$ des elektroaktiven Polymers nicht überschritten wird;

Relaxieren des gedehnten elektroaktiven Polymers von der maximalen Dehnung $\varepsilon_{max}$ bis zu der minimalen Dehnung $\varepsilon_{min}$ als Folge einer nachlassenden mechanischen Einwirkung der Wasserwelle,

wobei während des Relaxierens des elektroaktiven Polymers dieses während eines Zeitintervalls $\Delta t_{unload}$ von einem Zeitpunkt $t_2$ bis zu einem Zeitpunkt $t_{unload}$ bis auf eine Restladung $Q_0$ entladen wird; und

wobei zur Berechnung der elektrischen Zielladung erforderliche Zahlenwert der maximalen Dehnung $\varepsilon_{max}$ im Voraus abgeschätzt wird durch:

Abschätzen der maximalen Wellenhöhe $h_{max}$ einer Wasserwelle und
Korrelieren der maximalen Wellenhöhe $h_{max}$ mit der maximalen Dehnung $\varepsilon_{max}$ über eine Zuordnungsvorschrift.

2. Verfahren gemäß Anspruch 1, wobei der Zeitpunkt $t_1$ berechnet wird gemäß $t_1 = \dfrac{\lambda}{4 \cdot c} - \Delta t_{load}$, der Zeitpunkt $t_2$ berechnet wird gemäß $t_2 = 3 \cdot \dfrac{\lambda}{4 \cdot c}$, wobei weiterhin zu dem Zeitpunkt $t_{load}$ das elektroaktive Polymer die maximale Dehnung $\varepsilon_{max}$ eingenommen hat und zu dem Zeitpunkt $t_{unload}$ das elektroaktive Polymer die minimale Dehnung $\varepsilon_{min}$ eingenommen hat.

3. Verfahren gemäß Anspruch 2, wobei die Wellenlänge $\lambda$ ermittelt wird, indem mittels eines Beschleunigungssensors die vertikale Beschleunigung der Wasseroberfläche gemessen wird und:

die Oszillation des erhaltenen Beschleunigungssensor-Signals über eine Phasenregelschleife eine wellenlängenabhängige Spannung ergibt; und/oder
die Oszillation des erhaltenen Beschleunigungssensor-Signals über eine Fourier-oder Cosinus-Transformation in die Domäne c/$\lambda$, überführt wird.

4. Verfahren gemäß Anspruch 2, wobei die Wellenlänge $\lambda$ ermittelt wird, indem die zwischen den Elektroden am elektroaktiven Polymer anliegende Spannung gemessen wird und:

die Oszillation der Spannung über eine Phasenregelschleife eine wellenlängenabhängige Spannung ergibt; und/oder
die Oszillation der Spannung über eine Fourier- oder Cosinus-Transformation in die Domäne c/$\lambda$ überführt wird.

5. Verfahren gemäß Anspruch 1, wobei für den Fall, dass die tatsächliche Wellenhöhe sich gegenüber der geschätzten Wellenhöhe um so viel höher ist, dass mit der aufgebrachten Ladung die Durchschlagfeldstärke des elektroaktiven Polymers überschritten wird, aus dem elektroaktiven Polymer Ladung entnommen wird.

6. Verfahren gemäß Anspruch 1, wobei die Vorrichtung:

eine Boje;
eine axial segmentierte Kette von fluidgefüllten Körpern aus einem ein elektroaktives Polymer umfassenden Material; oder
eine Anordnung von durch ein Drehgelenk miteinander verbundenen Schwimmkörpern mit exzentrisch angeordneten vorgespannten Abschnitten aus ein elektroaktives Polymer umfassendem Material, welche sich bei einer Knickbewegung der Schwimmkörper gegensätzlich zueinander dehnen und relaxieren;
ist.

**7.** Verfahren gemäß Anspruch 1, wobei das elektroaktive Polymer in der Vorrichtung ein Polyurethan umfasst, welches erhältlich ist aus der Reaktion eines isocyanatfunktionellen Prepolymers mit einem Polyetherpolyol und wobei das Prepolymer erhältlich ist aus der Reaktion eines Polyisocyanats mit einem mindestens trifunktionellen Polyetherpolyol, welcher eine Molekülmasse $M_n$ von ≥6000 g/mol aufweist.

**8.** System zur Gewinnung von elektrischer Energie aus der Bewegungsenergie von Wasserwellen und geeignet zur Durchführung des Verfahrens gemäß Anspruch 1, umfassend:

eine Vorrichtung, welche so eingerichtet ist, dass beim Platzieren der Vorrichtung im Wasser ein Teil der Vorrichtung oberhalb der Wasseroberfläche angeordnet ist, wobei die Vorrichtung ein elektroaktives Polymer umfasst, welches zwischen Elektroden angeordnet ist und wobei in der Vorrichtung mechanische Energie aus einer Veränderung des Wasserspiegels auf das elektroaktive Polymer übertragen wird;
eine Sensoreinheit zur Gewinnung von Sensordaten über die Wasserwellen und/oder das elektroaktive Polymer;
eine Leistungseinheit zum Beaufschlagen des elektroaktiven Polymers mit elektrischer Ladung und zum Entladen des elektroaktiven Polymers; und
eine Steuereinheit, welche eingerichtet ist, um empfangene Daten aus der Sensoreinheit gemäß vorher definierter Datenmodelle in einem Berechnungsalgorithmus zu verarbeiten und als Ergebnis der Berechnung die Leistungselektronik anzusteuern, wobei weiterhin der Berechnungsalgorithmus die Abschätzung der maximalen Wellenhöhe von zukünftigen bei der Vorrichtung eintreffenden Wasserwellen umfasst.

**9.** System gemäß Anspruch 8, wobei die Sensoreinheit einen von der Vorrichtung beabstandeten Beschleunigungssensor umfasst.

**10.** System gemäß Anspruch 8, wobei die Sensoreinheit die zwischen den Elektroden am elektroaktiven Polymer anliegende Spannung und den Abstand der Elektroden am elektroaktiven Polymer misst.

**11.** Computerprogrammprodukt, umfassend durch einen Mikroprozessor ausführbare Befehle zur Durchführung der Berechnungen in dem Verfahren gemäß Anspruch 1.

FIG. 1

FIG. 2

FIG. 3

Europäisches
Patentamt

European
Patent Office

Office européen
des brevets

**EUROPÄISCHER RECHERCHENBERICHT**

**Nummer der Anmeldung**

EP 09 00 8697

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2008/267712 A1 (JEAN PHILIPPE F [FR] ET AL) 30. Oktober 2008 (2008-10-30) * das ganze Dokument * ----- | 1-11 | INV. F03B13/14 E02B9/08 G01N33/574 |
| A | US 2004/061338 A1 (WOODBRIDGE THOMAS C [US]) 1. April 2004 (2004-04-01) * das ganze Dokument * ----- | 1-11 | |
| A | WO 99/28623 A (DICK WILLIAM [IE]) 10. Juni 1999 (1999-06-10) * das ganze Dokument * ----- | 1-11 | |
| A | WO 96/25769 A (OCEAN POWER TECHNOLOGIES INC [US]) 22. August 1996 (1996-08-22) * das ganze Dokument * ----- | 1-11 | |

RECHERCHIERTE SACHGEBIETE (IPC)

F03B
E02B
G01N

Der vorliegende Recherchenbericht wurde für alle Patentansprüche erstellt

| Recherchenort | Abschlußdatum der Recherche | Prüfer |
|---|---|---|
| München | 17. August 2009 | Giorgini, Gabriele |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................
& : Mitglied der gleichen Patentfamilie, übereinstimmendes Dokument

EPO FORM 1503 03.82 (P04C03)

EP 2 282 048 A1

**ANHANG ZUM EUROPÄISCHEN RECHERCHENBERICHT
ÜBER DIE EUROPÄISCHE PATENTANMELDUNG NR.**

EP 09 00 8697

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-08-2009

| Im Recherchenbericht angeführtes Patentdokument | Datum der Veröffentlichung | Mitglied(er) der Patentfamilie | Datum der Veröffentlichung |
|---|---|---|---|
| US 2008267712 A1 | 30-10-2008 | WO 2008133774 A1 | 06-11-2008 |
| US 2004061338 A1 | 01-04-2004 | KEINE | |
| WO 9928623 A | 10-06-1999 | AU 749472 B2 | 27-06-2002 |
| | | AU 1444999 A | 16-06-1999 |
| | | DE 69817608 D1 | 02-10-2003 |
| | | EP 1036274 A1 | 20-09-2000 |
| | | ES 2205582 T3 | 01-05-2004 |
| | | NO 20002794 A | 31-07-2000 |
| | | NZ 505410 A | 01-02-2002 |
| | | PT 1036274 E | 30-01-2004 |
| | | US 6392314 B1 | 21-05-2002 |
| WO 9625769 A | 22-08-1996 | AU 4951996 A | 04-09-1996 |
| | | US 5578889 A | 26-11-1996 |

EPO FORM P0461

Für nähere Einzelheiten zu diesem Anhang : siehe Amtsblatt des Europäischen Patentamts, Nr.12/82

**EP 2 282 048 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 200106575 A1
- WO 2007130252 A2